# EUROPÄISCHE PATENTANMELDUNG

(11) **EP 2 462 961 A2**
(43) Veröffentlichungstag der Anmeldung: **13.06.2012**
(21) Anmeldenummer: 11189694.0
(22) Anmeldetag: 18.11.2011
(51) Int. Cl.: A61L 31/02, A61L 31/10, A61L 31/14, A61L 31/16

(54) **Implantat aus einem biokorrodierbaren Werkstoff und mit einer einen Gewebekleber enthaltenden Beschichtung**

(30) Priorität: 08.12.2010 US 420808 P
(71) Anmelder: Biotronik AG, 8180 Bülach (CH)
(72) Erfinder: Klocke, Björn, 8006 Zürich (CH); Gratz, Matthias, 91054 Erlangen (DE)
(74) Vertreter: Lindner-Vogt, Karin L.

(57) **Zusammenfassung**

Die Erfindung betrifft ein Implantat mit einem Grundkörper aus einem biokorrodierbaren Werkstoff. Eine Oberfläche des Implantats weist zumindest bereichsweise eine Beschichtung auf, die aus einem Gewebekleber besteht oder diesen enthält.

## Beschreibung

Die Erfindung betrifft ein beschichtetes Implantat mit einem Grundkörper aus einem biokorrodierbaren Werkstoff.

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, z.B. Stents), zur Befestigung und temporären Fixierung von Gewebeimplantaten und Gewebstransplantationen, aber auch zu orthopädischen Zwecken, zum Beispiel als Nagel, Platte oder Schraube. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch A-neurysmenstents bekannt, die vorrangig zur Abdichtung des Aneuryrismas dienen. Die Stützfunktion ist zusätzlich gegeben.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Das Implantat, insbesondere der Stent, besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare (hier als biokorrodierbar bezeichnete) Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder Ti-A16Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Die vorliegende Erfindung befasst sich mit biokorrodierbaren Implantatwerkstoffen, insbesondere Basislegierungen des Magnesiums.

Nach Implantation von biodegradierbaren Implantaten, insbesondere Stents, besteht das Risiko, dass unvollständig degradierte Fragmente oder ggf. nicht degradable Bestandteile wie Röntgenmarker unerwünschte Folgereaktionen auslösen. Diese Fragmente können beispielsweise beim Stent in den Blutstrom fallen und zu Embolien führen. Dies ist insbesondere dann der Fall, wenn der Stent noch nicht hinreichend in die Gefäßwand eingewachsen ist. Gerade bei Implantaten mit einer antiproliferativen Beschichtung kann dieses Problem vermehrt auftreten.

Degradable Stents aus Magnesiumlegierungen, aber auch aus polymeren Wirkstoffen sind typischerweise nicht röntgensichtbar. Deshalb sollten Röntgenmarker am Stent befestigt werden. Am einfachsten sind permanente Marker aus Gold oder Tantal, die ― gegebenenfalls elektrisch isoliert ― am Stent befestigt werden. Nach der Degradation des Magnesiumstents werden diese Marker jedoch nicht mehr mechanisch vom Stent an die Gefäßwand gepresst. Hier besteht die Gefahr, dass sich der Marker in dem Blutstrom (Lumen) ablöst und ebenfalls distal in der betreffenden Arterie zu Embolien führt.

Aus den genannten klinischen und regulatorischen Gründen sollte daher dafür gesorgt werden, dass potentiell auftretende Fragmente des biokorrodierbaren Implantats (hervorgehend aus Grundkörper, Marker oder anderen Zusatzelementen mit vom Grundkörper abweichenden Degradationseigenschaften) am Implantationsort, d. h. bei Stents an der Gefäßwand, verbleiben. Gerade bei wirkstoffbeschichteten degradablen Stents, bei denen durch den Einsatz antiproliferativer Medikamente die Einheilung verzögert beziehungsweise gestört wird, ist die Gefahr durch Fragmentbildung erhöht.

Ein oder mehrere der zuvor angesprochenen Nachteile des Standes der Technik werden mit Hilfe des erfindungsgemäßen Implantats mit einem Grundkörper aus einem biokorrodierbaren Werkstoff gelöst oder zumindest gemindert. Eine Oberfläche des Implantats weist zumindest bereichsweise eine Beschichtung auf, die aus einem Gewebekleber besteht oder diesen enthält.

Der Erfindung liegt die Erkenntnis zugrunde, dass durch Verwendung eines medizinischen Gewebeklebers der Grundkörper und/oder andere Zusatzelemente mit vom Grundkörper abweichenden Degradationseigenschaften sicher an das Gewebe am Implantationsort gebunden werden können. Der Gewebekleber kann dabei auf der gesamten Oberfläche des Implantats oder auch nur in Teilbereichen der Oberfläche des Implantats aufgetragen sein

Als Gewebekleber kommen alle bekannten medizinischen Gewebekleber in Frage. Insbesondere kann der Gewebekleber ein Cyanoacrylsäureester-Klebstoff oder ein Fibrinkleber sein.

Geeignete Cyanoacrylsäureester-Klebstoffe sind beispielsweise in DE 10 2007 003 768 A1 und US 6,224,622 B1 beschrieben. Die Wirkung Cyanoacrylsäure-basierter Klebstoffe beruht auf der Polymerisation von Cyanoacrylsäuremonomeren zu polymeren und/oder copolymeren Strukturen.

Die für erfindungsgemäße Lösung bevorzugten Cyanoacrylsäureester-Klebstoffe sind über den Verlauf von Tagen, Wochen oder Monaten selbst biodegradierbar.

Zur Bildung geeigneter Cyanoacrylsäureester-Klebstoffe kommen daher unter anderem Copolymere aus folgenden Cyanoacrylat-Monomeren und nicht-Cyanoacrylat-Monomeren in Frage: Cyanoacrylat-Monomere: Alkylcyanoacrylate, Alkenylcyanoacrylate, Alkoxyalkylcyanoacrylate; nicht-Cyanoacrylat-Monomere: Glycolyde, Lactide und Caprolactone.

Auch andere Monomere gelten als erfindungsgemäß besonders bevorzugt, solange sie zur Bildung biodegradierbarer, polymerer Strukturen geeignet sind.

Weiterhin bevorzugt liegt der Cyanoacrylsäureester-Klebstoffe als ein Polymer von Cyanoacrylat-Monomeren vor.

In einer besonders bevorzugten Ausführungsform, besonders im Fall der Fixierung am Ort der Implantation nichtdegradierbarer Bestandteile von wenigstens in Teilen degradierbaren Implantaten, ist auch die Verwendung nicht oder nur sehr langsam biodegradierbarer Cyanoacrylsäureester-Klebstoff im Sinne dieser Erfindung geeignet, z.B. Cyanoacrylat-Polymere aus Monomeren mit langen organischen Ketten, wie z.B. bei Octyl-cyanoacrylate. Das Klebstoffmaterial verbleibt in dieser Anwendung ebenso wie die nichtdegradierbaren Implantatbestandteile am Ort der Implantation zurück.

Geeignete Fibrinkleber im Rahmen der vorliegenden Erfindung sind Zweikomponentensysteme, die bei Vermischung der Komponenten zeitnah stark haftvermittelnd wirken. Für die Verwendung in Beschichtungen dürfen daher die dem Fachmann bekannten Inhaltsstoffe nicht in bereits vermischter Form vorliegen. Die erfindungsgemäße Beschichtung stellt eine der beiden Komponenten des Gewebeklebers bereit. Die Verwendbarkeit von Fibrinklebern in Implantatbeschichtungen basiert hier auf dem Fakt, dass das üblicherweise in einer der Komponenten des Fibrinklebers enthaltene Fibrinogen entgegen der dem Fachmann bekannten Ausführungen nicht Bestandteil einer erfindungsgemäßen Beschichtung ist, da es durch die Verwendung des Implantats in Blutgefäßen ohnehin bereits am Implantationsort vorliegt. In einer Ausführungsform der erfindungsgemäßen Beschichtung liegt Aprotinin gemeinsam mit den anderen üblichen Bestandteilen von Fibrinklebern, einschließlich Faktor XIII, Thrombin und einer Calciumquelle wie z.B. Calciumchlorid, vor. Erst durch die Rekrutierung zirkulierenden Fibrinogens nach der Implantation wird die Verklebung gewährleistet.

Eine Beschichtung im Sinne der Erfindung ist eine zumindest abschnittsweise Auftragung der Komponenten der Beschichtung auf den Grundkörper, Röntgenmarker und/oder weitere Strukturelemente des Implantats. Vorzugsweise ist im Falle eines Stents die Beschichtung nur abluminal auf dem Implantat aufgetragen. Eine Schichtdicke liegt vorzugsweise im Bereich von 1 nm bis 100 µm, besonders bevorzugt 300 nm bis 15 µm. Die erfindungsgemäße Beschichtung kann direkt auf die Implantatoberfläche aufgetragen werden oder es sind weitere Zwischenschichten vorgesehen; so kann gegebenenfalls der Grundkörper des Implantats eine anorganische Grundschicht aufweisen, die die Haftung der erfindungsgemäßen Beschichtung verbessert. Auf die erfindungsgemäße Beschichtung können weitere Schichten aufgetragen werden, um zum Beispiel die Einführung des Implantats in den Körper zu erleichtern. Verfahren zur Beschichtung von Implantaten sind dem Fachmann bekannt (z.B. Aufpipettieren, Aufrollen).

Als biokorrodierbar im Sinne der Erfindung werden Werkstoffe bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Vorzugsweise ist der biokorrodierbare Implantatwerkstoff eine biokorrodierbare Metalllegierung oder ein biodegradierbares Polymer.

Vorzugsweise ist der biokorrodierbare Implantatwerkstoff eine biokorrodierbare Magnesiumlegierung, eine Eisenlegierung oder ein biokorrodierbares Polylactid, insbesondere PLLA. Unter Magnesiumlegierung wird vorliegend ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/1, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C und pH 7,38 gelagert. In zeitlichen Abständen ― abgestimmt auf das zu erwartende Korrosionsverhalten ― von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

In einer besonderen Ausführungsform kann das Implantat Zusatzelemente wie beispielsweise einen permanenten oder biodegradierbaren Röntgenmarker aufweisen. Entsprechende Röntgenmarker sind im Stand der Technik bekannt und können unter anderem auf Basis chemischer Elemente mit hohen Ordnungszahlen, wie Tantal, oder ionischer oder nicht-ionischer Komplexe, sowie auf Basis von Polymeren enthaltend z.B. Iod oder Gadolinium hergestellt sein. Beispielsweise kann am Grundkörper eines biokorrodierbaren Implantats ein permanenter Röntgenmarker fixiert sein. Auf diesem Implantat kann anschließend die erfindungsgemäße Beschichtung aufgetragen werden. Vorzugsweise ist die den Gewebekleber enthaltende Beschichtung nur auf dem Röntgenmarker aufgetragen. Weiterhin bevorzugt wird der permanente Röntgenmarker mit einem nicht biodegradierbarer Cyanoacrylsäurekleber beschichtet. Diese Vorrichtungen haben den Vorteil, dass das Klebstoffmaterial ebenso wie die nichtdegradierbaren Implantatbestandteile am Ort der Implantation zurückbleibt. Somit lassen sich Nebenwirkungen, hervorgerufen durch nicht degradierbare Elemente des Implantats, verhindern.

Die Beschichtung kann einen oder mehrere Wirkstoffe enthalten, der nach der Implantation freigesetzt werden. Unter Wirkstoff im erfindungsgemäßen Sinne wird ein Arzneistoff mit pharmazeutischer Wirkung verstanden, der im menschlichen oder tierischen Körper zur Heilung, Linderung, Verhütung oder Erkennung von Krankheiten dient. Wirkstoffe umfassen insbesondere Paclitaxel, Sirolimus und dessen Derivate. Insbesondere sind Wirkstoffe vorteilhaft, die auf mTOR wirken, sowie RAS Inhibitoren, insbesondere solche, die die RAS-Adhäsion verhindern.

Der wirkstoffhaltige Teil der Beschichtung muss nicht identisch mit der Schicht sein, die den Gewebekleber enthält. So kann die Wirkstoff-enthaltende Schicht zusätzlich zum Gewebekleber aufgetragen werden. Diese Schicht kann unter und/oder über dem Gewebekleber aufgetragen werden.

In einer besonderen Ausführungsform ist mindestens eine, vorzugsweise abluminale Schicht, zum Beispiel zur Förderung der Endothelialisierung, denkbar.

In einer weiteren Ausführungsform liegt die Wirkstoff-enthaltende Schicht unter dem Gewebekleber, direkt auf der Implantatoberfläche vor. Diese Wirkstoffschicht stellt somit einen Haftvermittler (Primer) für den Gewebekleber auf der Implantatoberfläche dar. Dabei kann die Wirkstoff-enthaltende Beschichtung zusätzlich aufgeraut oder mit symmetrischen oder zufälligen Strukturelementen wie Einschnitte, Vertiefung (Rillen, Gruben), Erhebungen versehen sein.

Zur besseren Haftung des Gewebeklebers auf der Implantatoberfläche kann die Oberfläche des Implantats zusätzlich einer mechanischen und/oder chemischen Präparation unterzogen werden. So kann ein Haftvermittler (Primer), z.B. Silane, bevorzugt ein polymerer Haftvermittler, wie z.B. Parylene, auf die Implantatoberfläche beschichtet werden. Alternativ oder zusätzlich kann die Oberfläche des Implantats aufgeraut oder mit symmetrischen oder zufälligen Strukturelementen wie Einschnitte, Vertiefung (Rillen, Gruben), Erhebungen versehen werden.

Um einen Abrieb oder die Aktivierung der den Gewebekleber enthaltenden Beschichtung beim Einführen des Implantats zu verhindern, kann zum Beispiel ein Beschichtungsmaterial mit hoher Schichthaftung oder eine temporäre Deckschicht (Topcoat) aufgetragen werden. Alternativ kann ein Abrieb oder eine Aktivierung auch durch eine Hülse oder einen Schutzschlauch, der direkt vor dem Setzen des Implantats zurückgezogen wird, vermieden werden. Bei Verwendung wirkstoffeluierender Systeme kann insbesondere die Wirkstoff-enthaltende Schicht als temporäre Deckschicht für die den Gewebekleber enthaltende Schicht dienen. Dabei kann die Oberfläche der Deckschicht zusätzlich aufgeraut oder mit symmetrischen oder zufälligen Strukturelementen wie Einschnitte, Vertiefung (Rillen, Gruben), Erhebungen versehen sein.

Um die Lagerstabilität einer solchen erfindungsgemäßen Beschichtung zu erhöhen, wird das Implantat ab der Beschichtung und bis zur Verwendung vor einem Kontakt mit wässrigen Medien und hohen Luftfeuchtigkeiten geschützt. Dabei finden "trockene" Schutzgase als auch feuchtigkeitsdichte Verpackungen, z.B. auf Aluminium- oder Kunststoffbasis Verwendung.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Vorzugsweise ist das Implantat ein Stent.

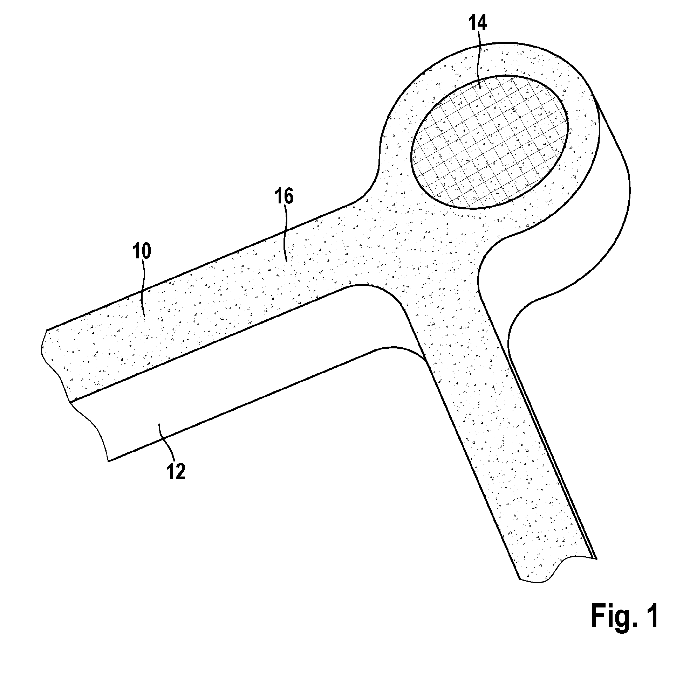

Die Erfindung wird nachfolgend anhand eines Ausführungsbeispiels und einer dazugehörig Figur näher erläutert. Die einzige Figur zeigt in einer schematischen Darstellung einen Stent mit der erfindungsgemäßen Beschichtung.

In der einzigen Figur ist ein Stent 10 mit einem Grundkörper 12 aus einer biokorrodierbaren Magnesiumlegierung und einem am Grundkörper 12 fixierten Röntgenmarker 14 dargestellt. Sowohl auf der abluminalen Seite des Grundkörpers 12, als auch des Röntgenmarkers 14 ist eine Beschichtung 16 (angedeutet durch die Punktierung) aufgetragen, die einen Gewebekleber, zum Beispiel einen Cyanacrylsäureester-Klebstoff, enthält.

## Patentansprüche

1. Implantat mit einem Grundkörper aus einem biokorrodierbaren Werkstoff, wobei eine Oberfläche des Implantats zumindest bereichsweise eine Beschichtung aufweist, die aus einem Gewebekleber besteht oder diesen enthält.

2. Implantat nach Anspruch 1, bei dem der Gewebekleber ein Cyanoacrylsäureester-Klebstoff oder ein Fibrinkleber ist.

3. Implantat nach Anspruch 1 oder 2, bei dem das Implantat einen Röntgenmarker aufweist, der zumindest bereichsweise die Beschichtung trägt.

4. Implantat nach einem der vorhergehenden Ansprüche, bei dem die Beschichtung mindestens einen Wirkstoff aufweist.

5. Implantat nach einem der vorhergehenden Ansprüche, bei dem zusätzlich eine Wirkstoff-enthaltende Beschichtung unter und/oder über dem Gewebekleber aufgetragen ist.

6. Implantat nach Anspruch 5, bei dem der Wirkstoff Paclitaxel, Sirolimus oder ein Derivat von Sirolimus ist.

7. Implantat nach Anspruch 5 oder 6, bei dem die Wirkstoff-enthaltende Beschichtung abluminal auf dem Implantat aufgetragen ist.

8. Implantat nach einem der vorhergehenden Ansprüche, bei dem auf der Implantatoberfläche ein Haftvermittler für den Gewebekleber beschichtet ist.

9. Implantat nach einem der vorhergehenden Ansprüche, bei dem die Implantatoberfläche zusätzlich aufgeraut oder mit symmetrischen oder zufälligen Strukturelementen wie Einschnitte, Vertiefung (Rillen, Gruben), Erhebungen versehen ist.

10. Implantat nach einem der vorherigen Ansprüche, bei dem das Implantat eine temporäre Deckschicht aufweist.

11. Implantat nach einem der vorherigen Ansprüche, bei dem eine Hülse oder ein Schutzschlauch auf der Oberfläche des Implantats aufgebracht wird.

12. Implantat nach einem der vorherigen Ansprüche, bei dem der Grundkörper aus einer biokorrodierbaren Magnesiumlegierung besteht.

13. Implantat nach einem der vorherigen Ansprüche, bei dem das Implantat ein Stent ist.
